# EUROPEAN PATENT SPECIFICATION

(11) **EP 4 188 511 B1**
(45) Date of publication and mention of the grant of the patent: **06.05.2026**
(21) Application number: 21759450.6
(22) Date of filing: 28.07.2021
(51) Int. Cl.: A61M 39/06

(54) **SECUREMENT DEVICES FOR INTRACARDIAC BLOOD PUMP SYSTEMS**
BEFESTIGUNGSVORRICHTUNGEN FÜR INTRAKARDIALE BLUTPUMPENSYSTEME
DISPOSITIFS DE FIXATION POUR SYSTÈMES DE POMPE À SANG INTRACARDIAQUES

(30) Priority: 29.07.2020 US 202063058003 P
(43) Date of publication of application: 07.06.2023
(73) Proprietor: Abiomed, Inc., Danvers, MA 01923 (US)
(72) Inventor: SHEILS, Christopher, W., Danvers, MA 01923 (US); RODRIGUES-BRIMMERS, Cristine, Gracinda, 52074 Aachen (DE); SCHAEFER, Robert, Danvers, MA 01923 (US)
(74) Representative: Klunker IP Patentanwälte PartG mbB
(86) International application number: PCT/US2021/043491
(87) International publication number: WO 2022/026569

(56) References cited:
- DE-A1- 4 213 691
- US-A- 5 921 968
- US-A1- 2019 070 401

## Description

### BACKGROUND

Intracardiac heart pump assemblies can be introduced into the heart either surgically or percutaneously and used to deliver blood from one location in the heart or circulatory system to another location in the heart or circulatory system. For example, when deployed in the heart, an intracardiac pump can pump blood from the left ventricle of the heart into the aorta, or pump blood from the inferior vena cava into the pulmonary artery. Intracardiac pumps can be powered by a motor located outside of the patient's body (and accompanying drive cable) or by an onboard motor located inside the patient's body. Some intracardiac blood pump systems can operate in parallel with the native heart to supplement cardiac output and partially or fully unload components of the heart. Examples of such systems include the IMPELLA^{®} family of devices (Abiomed, Inc., Danvers Mass.).

In one common approach, an intracardiac blood pump is inserted by a catheterization procedure through the femoral artery using a sheath, such as a peel away introducer sheath. The sheath can alternatively be inserted in other locations such as in the femoral vein or any path for delivery of a pump for supporting either the left or right side of the heart.

The introducer sheath can be inserted into the femoral artery through an arteriotomy to create an insertion path for the pump assembly. A portion of the pump assembly is then advanced through an inner lumen of the introducer and into the artery. In some cases, once the pump assembly has been inserted, a narrower repositioning sheath may be inserted into the introducer sheath, and the introducer sheath may be peeled away. The repositioning sheath may be equipped with a structure for securing the repositioning sheath assembly to the patient. For example, the proximal hub of the repositioning sheath assembly may have a butterfly structure that may be sutured to the skin of the patient.

In some cases, the introducer sheath may remain in place even after the pump assembly has been inserted. In such cases, a repositioning sheath may or may not be used in addition to the introducer sheath. Where a repositioning sheath is used, it may, for example, be inserted within the introducer sheath. In some cases, the repositioning sheath may be configured to fit within the lumen of the introducer sheath. In some cases, the lumen of the introducer sheath may be configured to expand to permit the repositioning sheath to be inserted therein. In some cases, the introducer sheath may be configured such that the repositioning sheath is not inserted into the lumen of the introducer sheath. Where the introducer sheath remains in place, it may also be equipped with a structure (e.g., a suture butterfly) for securing the introducer sheath assembly to the patient.

Regardless of whether an introducer sheath, repositioning sheath, or both remain in use after the insertion of the pump assembly, a securement device may be used to hold the catheter of the pump assembly in relation to the sheath(s) through which it has been inserted. For example, a Tuohy-Borst device may be attached or incorporated into to a hub of the introducer sheath or repositioning sheath. Turning a barrel of the Tuohy-Borst device in one direction compresses a deformable internal component (e.g., a deformable ring or sleeve), which can thus be used to form a seal against the catheter of the pump assembly and resist movement of the catheter within the Tuohy-Borst device. Turning the barrel of the Tuohy-Borst device in the other direction allows the deformable internal component to relax, thus permitting movement of the catheter. However, because a Tuohy-Borst device will not restrict movement of the catheter without a deliberate action by the operator, it is possible for it to be inadvertently left in an open or semi-open position. Likewise, because a Tuohy-Borst device can provide variable amounts of resistance, an operator may accidentally under-tighten it or it may be unintentionally loosed (e.g., due to movements by the patient), allowing the catheter to move after it has been placed in its desired location. Patent document DE4213691 discloses features falling under the preamble of claim 1.

### BRIEF SUMMARY

The present technology relates to improved securement devices for use with intracardiac blood pump assemblies. More particularly, the present technology provides securement devices that may be used with a sheath assembly (e.g., an introducer sheath assembly, repositioning sheath assembly), and which are configured to restrict movement of the catheter of the pump assembly within the sheath assembly except when a mechanism (e.g., button) of the securement device is actively being actuated (e.g., pressed, held, etc.). As will be explained in further detail below, the securement devices of the present technology may be provided as a modular unit that can be attached to a hub or a hemostasis valve of an introducer sheath or a repositioning sheath assembly. Likewise, the securement devices of the present technology may also be provided as an integral part of a hemostasis valve, or as an integral part of an introducer sheath assembly or a repositioning sheath assembly.

In one aspect, the disclosure describes a device for securing an intravenous medical device, comprising: (i) a flexible sleeve having a first lumen configured to receive at least a portion of the intravenous medical device; (ii) a button having a bore configured to receive at least a portion of the flexible sleeve; (iii) a spring element; and (iv) a housing comprising: a second lumen configured to house the flexible sleeve; and a cavity configured to house the button and the spring element, wherein the spring element is configured to exert a force on the button tending to cause the bore to compress the flexible sleeve unless the button is being held in a depressed state. In some aspects, the housing comprises at least one protrusion with at least one eyelet, the at least one protrusion configured to enable the housing to be sutured to a patient's skin. According to one variation of the invention, the housing further comprises a hemostasis valve. In some aspects, the housing is configured to couple with a hemostasis valve. According to one variation of the invention, the housing comprises at least one slot configured to accept at least one peg of the hemostasis valve when the housing is coupled with the hemostasis valve. In some aspects, the at least one slot and the at least one peg comprise a bayonet connection. In some aspects, the at least one slot further comprises at least one eyelet configured to engage with the at least one peg. In some aspects, the at least one slot further comprises at least one detent configured to engage with the at least one peg. In some aspects, the housing further comprises a seal configured to deform when the housing is coupled with the hemostasis valve.

In another aspect, the disclosure describes a sheath assembly, comprising: (a) a sheath body configured for insertion into a patient's vasculature, and to receive at least a portion of an intravenous medical device; (b) a sheath hub coupled to a proximal end of the sheath body; and (c) a securement device integral with the sheath hub or configured to couple with the sheath hub, the securement device comprising: (i) a flexible sleeve having a first lumen configured to receive at least a portion of the intravenous medical device; (ii) a button having a bore configured to receive at least a portion of the flexible sleeve; (iii) a spring element; and (iv) a housing comprising: a second lumen configured to house the flexible sleeve; and a cavity configured to house the button and the spring element, wherein the spring element is configured to exert a force on the button tending to cause the bore to compress the flexible sleeve unless the button is being held in a depressed state. In some aspects, the housing of the securement device comprises at least one protrusion with at least one eyelet, the at least one protrusion configured to enable the housing of the securement device to be sutured to a patient's skin. In some aspects, the sheath hub comprises at least one protrusion with at least one eyelet, the at least one protrusion configured to enable the sheath hub to be sutured to a patient's skin. In some aspects, the housing of the securement device further comprises a hemostasis valve. In some aspects, the sheath hub further comprises a hemostasis valve. In some aspects, the housing of the securement device is configured to couple with a hemostasis valve. In some aspects, the housing of the securement device comprises at least one slot configured to accept at least one peg of the hemostasis valve when the housing of the securement device is coupled with the hemostasis valve. In some aspects, the at least one slot and the at least one peg comprise a bayonet connection. In some aspects, the at least one slot further comprises at least one eyelet configured to engage with the at least one peg. In some aspects, the at least one slot further comprises at least one detent configured to engage with the at least one peg. In some aspects, the housing of the securement device further comprises a seal configured to deform when the housing of the securement device is coupled with the hemostasis valve.

### BRIEF DESCRIPTION OF DRAWINGS

FIG. 1 shows an exemplary system including a blood pump and repositioning sheath assembly, and an introducer sheath assembly, in accordance with aspects of the disclosure;
FIG. 2 shows an enlarged view of the blood pump and repositioning sheath assembly of FIG. 1;
FIG. 3 shows an enlarged view of a blood pump and repositioning sheath assembly using an exemplary button-actuated securement device, in accordance with aspects of the disclosure;
FIG. 4 shows an isometric view of a three-dimensional model of the exemplary button-actuated securing device of FIG. 3;
FIG. 5 shows a line-drawing of the exemplary button-actuated securing device of FIG. 3;
FIG. 6 shows an exploded view of the exemplary button-actuated securing device of FIG. 3;
FIG. 7 shows a cross-sectional profile view of the exemplary button-actuated securing device of FIG. 3;
FIG. 8 shows an enlarged view of a blood pump and repositioning sheath assembly using an exemplary button-actuated securement device, in accordance with aspects of the disclosure;
FIG. 9 shows an enlarged view of a blood pump and repositioning sheath assembly using an exemplary button-actuated securement device, in accordance with aspects of the disclosure; and
FIGS. 10A-10D are exploded views of features of the apparatus of FIG. 6 but with a different retention pin design and a different design for the second bore of the button.

### DETAILED DESCRIPTION

Embodiments of the present disclosure are described in detail with reference to the figures wherein like reference numerals identify similar or identical elements. It is to be understood that the disclosed embodiments are merely examples of the disclosure, which may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

To provide an overall understanding of the systems, methods, and devices described herein, certain illustrative embodiments will be described. Although the embodiments and features described herein are specifically described for use in connection with an intracardiac heart pump system, it will be understood that all the components and other features outlined below may be combined with one another in any suitable manner and may be adapted and applied to other types of medical devices such as electrophysiology study and catheter ablation devices, angioplasty and stenting devices, angiographic catheters, peripherally inserted central catheters, central venous catheters, midline catheters, peripheral catheters, inferior vena cava filters, abdominal aortic aneurysm therapy devices, thrombectomy devices, TAVR delivery systems, cardiac therapy and cardiac assist devices, including balloon pumps, cardiac assist devices implanted using a surgical incision, and any other venous or arterial based introduced catheters and devices.

The systems, methods, and devices described herein provide securement devices that may be used with a sheath assembly (e.g., an introducer sheath assembly, repositioning sheath assembly), and which are configured to restrict movement of the catheter of the pump assembly within the sheath assembly except when a mechanism (e.g., button) of the securement device is actively being actuated (e.g., pressed, held, etc.). In that regard, the securement devices of the present technology can be configured to permit movement of the catheter only when a mechanism (e.g., button) is depressed, and to automatically return to a secured or "locked" state upon release of the mechanism. Such a configuration beneficially prevents an operator from forgetting to secure the blood pump assembly after it has been positioned in the patient, and reduces the risk that the securement device may be unintentionally unlocked (e.g., due to movements by the patient). In addition, by providing full resistance in its resting state, the securement devices of the present technology prevent an operator from accidentally under-tightening the securement device, as may occur for example with Tuohy-Borst devices.

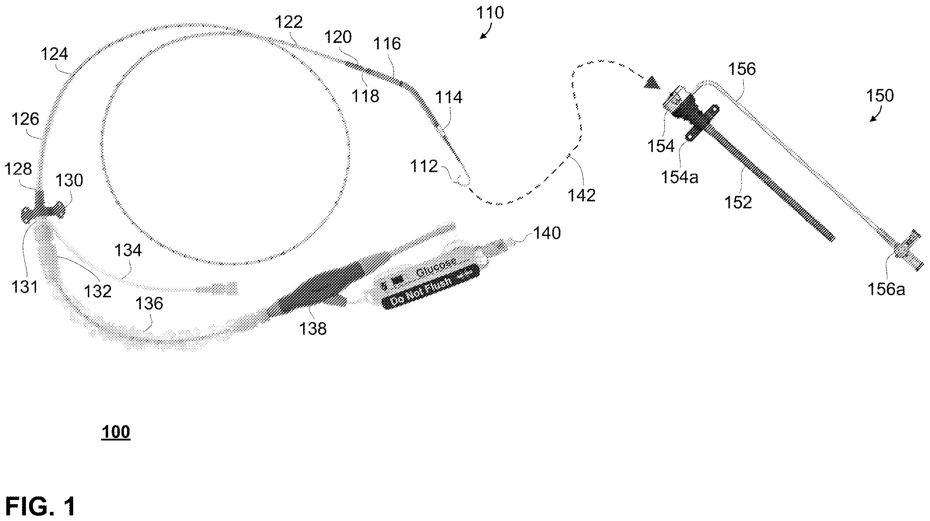

FIG. 1 shows an exemplary system 100 comprising a blood pump and repositioning sheath assembly 110, and an introducer sheath assembly 150, in accordance with aspects of the disclosure.

The exemplary blood pump and repositioning sheath assembly 110 includes a handle 138 at its proximal end. Handle 138 is coupled to the proximal end of catheter 122. In addition, in the example of FIG. 1, a purge fluid port 140 is connected to handle 138 in order to provide purge fluid (e.g., a solution of dextrose or glucose with heparin) to a purge lumen (not shown) within catheter 122. The purge lumen of catheter 122 is configured to deliver purge fluid to the motor housing 120.

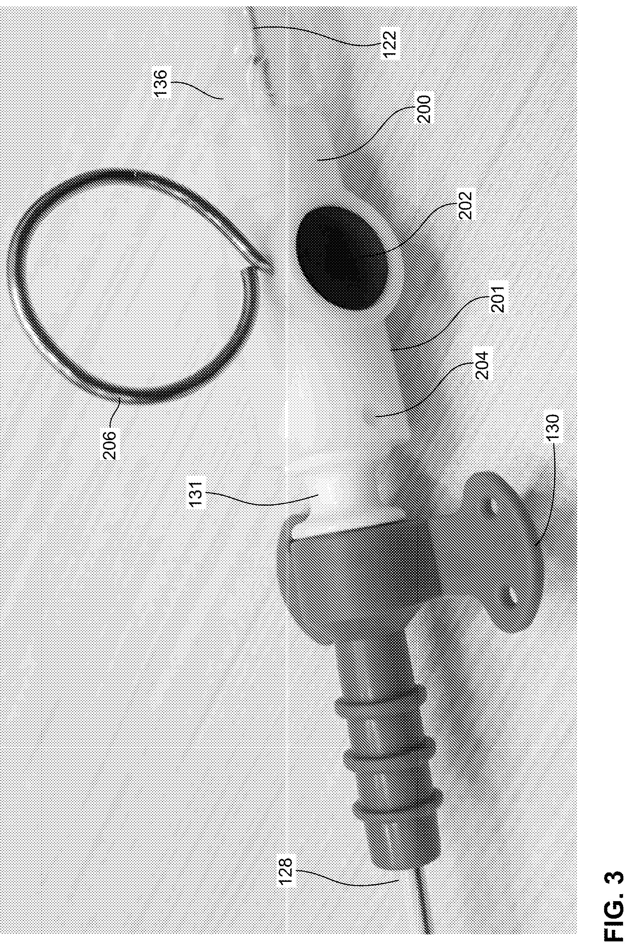

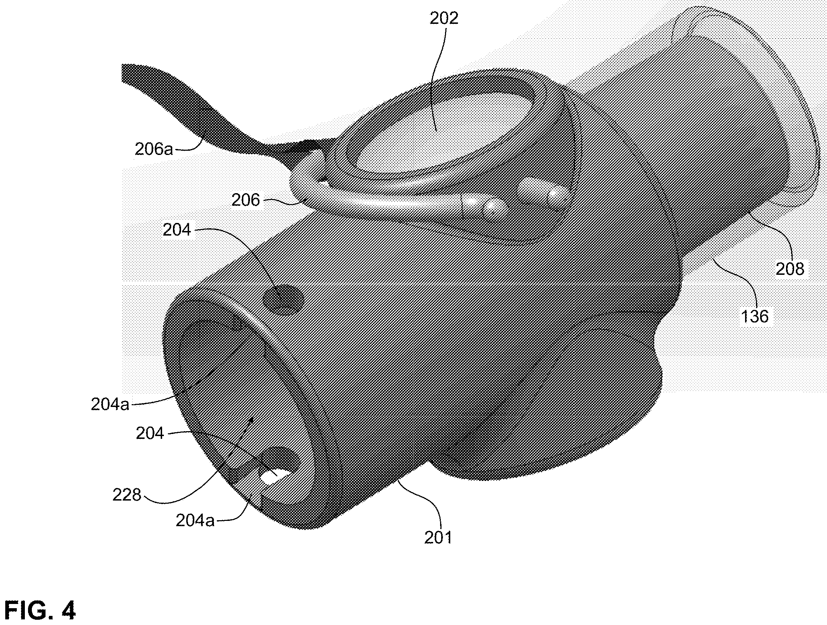

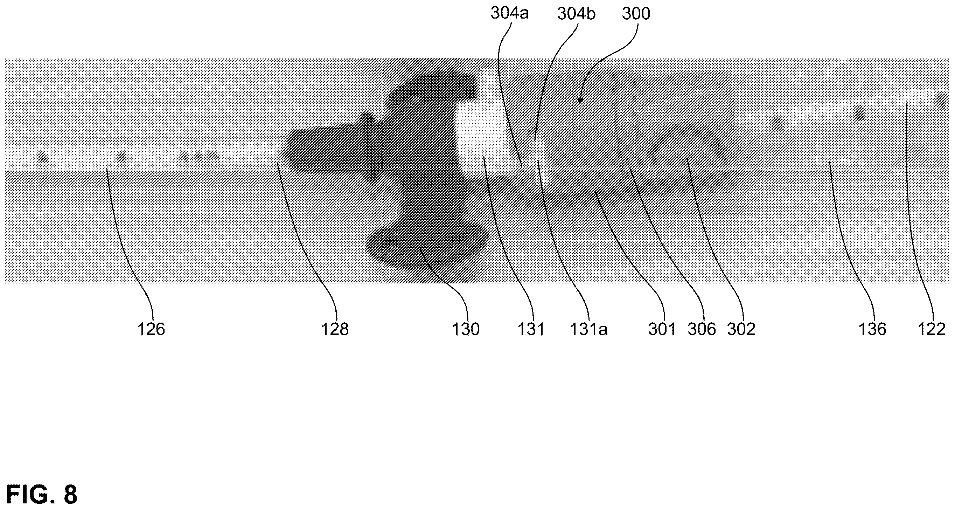

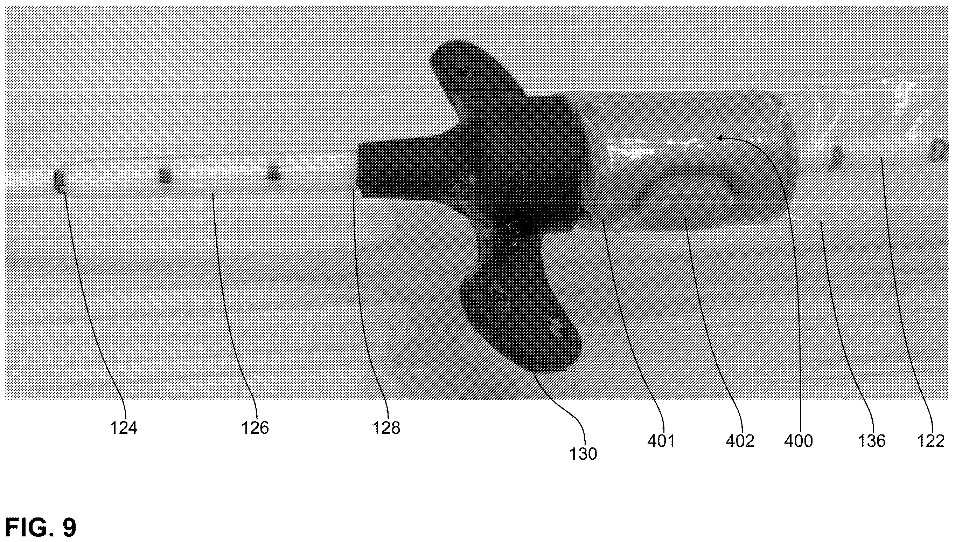

Between handle 138 and securement device 132, the catheter 122 is enclosed within a protective sleeve 136. Protective sleeve 136 is configured to prevent contamination of catheter 122 as it is advanced in the distal direction for insertion into the patient's vasculature. Protective sleeve 136 may be comprised of any suitable material, and may be secured at its proximal and distal ends in any suitable manner. The distal end of protective sleeve 136 is coupled to securement device 132. In that regard, for the button-actuated securement devices of the present technology, the protective sleeve 136 may be coupled proximally of the button (e.g., as shown in FIGS. 3 and 4), or may be coupled distally of the button so that the button is maintained within protective sleeve 136 and contamination around the edges of the button is avoided (e.g., as shown in FIGS. 8 and 9). In the example of FIG. 1, securement device is coupled at its distal end to a hemostasis valve 131, which in turn is attached to butterfly 130. Hemostasis valve 131 may be integrated with butterfly 130, or may be removably coupled thereto. In addition, as discussed above and below, hemostasis valve 131 and securement device 132 may be incorporated into a single unit. Butterfly 130 is coupled at its distal end to the proximal end 128 of repositioning sheath 126. The distal end of repositioning sheath 126 is indicated with reference numeral 124. Repositioning sheath 126 is configured with a lumen sized to allow passage of at least catheter 122, but otherwise may have any suitable length and construction.

The distal end of catheter 122 is connected to the proximal end of motor housing 120. In some aspects of the technology, motor housing 120 may include a motor and impeller. In other aspects of the technology, the motor may be external to the patient, in which case catheter 122 may enclose a flexible drive shaft/cable, and motor housing 120 may enclose an impeller connected to that drive shaft/cable. As will be appreciated, the securement devices of the present technology may be used with any intracardiac blood pump or other intravenous medical device for which an operator wishes to restrict movement.

In the example of FIG. 1, the distal end of motor housing 120 is coupled to a blood outflow cage 118. The distal end of blood outflow cage 118 is coupled to a cannula 116, which in turn is coupled at its distal end to a blood inflow cage 114. When the pump is being operated, blood will be pumped in the proximal direction from blood inflow cage 114, through cannula 116, to blood outflow cage 118. However, in some aspects of the technology, the pump may instead be configured to pump blood in the distal direction (e.g., for applications where the pump is used for right heart support), in which case element 118 would operate as the blood inflow cage, and element 114 would operate as the blood outflow cage. Finally, in some aspects of the technology, the distal end of blood inflow cage 114 may include an atraumatic extension such as the pigtail extension 112 shown in the example of FIG. 1.

Generally, a blood pump will initially be inserted into the patient's vasculature via an introducer sheath such as the exemplary introducer sheath assembly 150. In that regard, introducer sheath assembly 150 comprises an introducer sheath body 152, a hub 154, and an irrigation line 156. Introducer sheath body 152 may be any suitable type of sheath. For example, in some aspects of the technology, introducer sheath body 152 may be a tear-away sheath configured to be ripped or peeled along its length and discarded after the repositioning sheath 126 has been inserted into the patient's vasculature. However, in other aspects of the technology, introducer sheath body 152 may be an expandable sheath configured to stretch to allow the passage of the largest portions of the blood pump, and to contract thereafter, in which case the introducer sheath may remain in place even after the pump has been inserted into the patient's vasculature. In the example of FIG. 1, hub 154 is shown with an optional butterfly 154a, which may be used to secure the introducer sheath assembly 150 to the patient (e.g., using adhesives or sutures). Hub 154 may further include a hemostasis valve (not shown). In addition, irrigation line 156 is shown with an optional stopcock assembly 156a, which in the example of FIG. 1 includes two ports.

Once the blood pump has been inserted into the patient's vasculature, the operator may advance the blood pump to its desired location in the body (e.g., left heart or right heart). As the catheter 122 advances further into the patient's vasculature, the distal end 124 of repositioning sheath 126 may be inserted into introducer sheath assembly 150. In this regard, introducer sheath body 152 will act as the conduit for repositioning sheath 126 to enter the patient's vasculature. In other cases, as the catheter 122 is advanced into the patient's vasculature, the repositioning sheath 126 may remain outside of introducer 150. As already noted, where introducer sheath assembly 150 is a tear-away design, after repositioning sheath 126 has been inserted into the patient's vasculature, the introducer sheath assembly 150 may be removed by breaking hub 154 and tearing introducer sheath body 152 along its length. However, where introducer sheath assembly 150 is an expandable design, it may remain in the body, either surrounding some or all of the repositioning sheath 126, or, in cases where the repositioning sheath 126 remains outside of the body, surrounding catheter 122.

Once repositioning sheath 126 has been fully inserted, the operator may secure it to the patient at or near the insertion site using butterfly 130. In that regard, butterfly 130 may be affixed to the patient (e.g., using adhesives or sutures) in order to secure the repositioning sheath 126 and securement device 132 relative to the patient. Alternatively, if repositioning sheath 126 remains outside of the body, the introducer sheath 150 may be affixed to the patient using butterfly 154a, and a securement device similar to 132 could be located at the introducer hub 154, and similarly allow for pump securement relative to the patient. Thereafter, once the blood pump has been advanced to the desired location within the patient's body, the operator may use securement device 132 to restrict further movement of the blood pump within the patient. In that regard, securement device 132 may be any device suitable for optionally allowing and restricting movement of catheter 122 therethrough. Although the specific securement device 132 depicted in the exemplary system 100 of FIG. 1 is a conventional Tuohy-Borst type device, any of the improved securement devices depicted and described with respect to FIGS. 3-9 may be used in place of securement device 132. In addition, although the securement devices of FIGS. 1-8 are shown as modular units that couple to a hemostasis valve of a repositioning sheath assembly, the securement devices of the present technology may alternatively be provided as: (1) a modular unit that couples to a hemostasis valve of an introducer sheath assembly; (2) a modular unit that incorporates a hemostasis valve and couples to a hub of a repositioning sheath assembly or an introducer sheath assembly; or (3) as an integral part of a repositioning sheath assembly or an introducer sheath assembly.

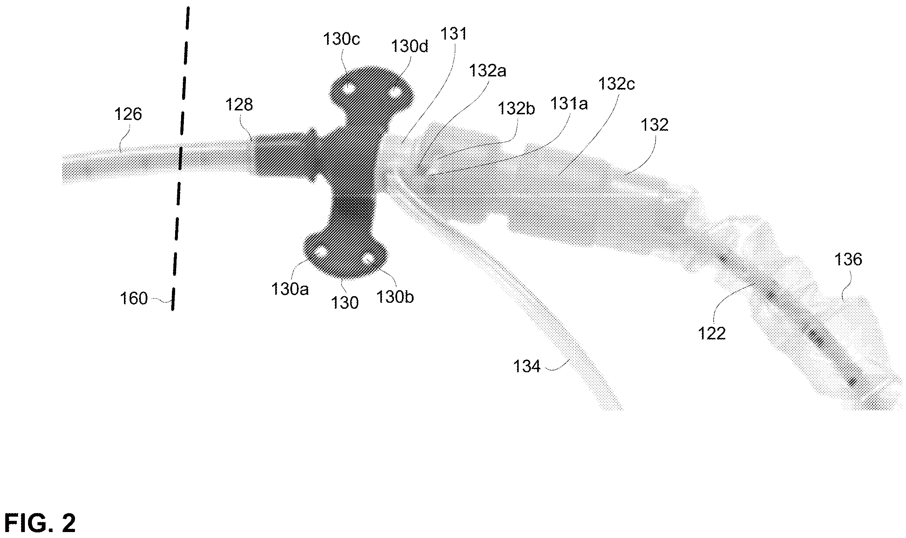

FIG. 2 shows an enlarged view of the blood pump and repositioning sheath assembly of FIG. 1. In that regard, all reference numerals shared between FIGS. 1 and 2 are meant to indicate the same features, and will not be addressed again except as necessary to explain the additional features shown in FIG. 2. FIG. 2 reproduces a portion of blood pump and repositioning sheath assembly 110, oriented with its distal end to the left as though repositioning sheath 126 has been passed through a patient's skin surface (indicated by dashed line 160) from right to left. Oriented in this way, the proximal end 128 of repositioning sheath 126 would remain on the outside of the patient's body, and butterfly 130 may be secured to the patient's skin using sutures passed through suture eyelets 130a, 130b, 130c, and 130d.

The enlarged view of FIG. 2 shows a bayonet connection between hemostasis valve 131 and securement device 132. In that regard, the distal end of securement device 132 is configured such that it can be coupled to a proximal end of hemostasis valve 131 by pushing the two parts together and turning them relative to one another. In the example of FIG. 2, the distal portion of securement device 132 has a cylindrical collar with one or more slots, and the proximal portion of hemostasis valve 131 has a cylindrical projection with one or more pegs 131a. When the cylindrical collar of securement device 132 is advanced over the cylindrical projection of hemostasis valve 131, each peg 131a will enter one of the slots in securement device 132 at an entrance point 132a. Then, by rotating the securement device 132 relative to hemostasis valve 131, each peg 131a will move toward an end point 132b of the slot, thus preventing securement device 132 from being pulled away from hemostasis valve 131 (without first rotating the two components in the opposite direction). In addition, the internal interface between the cylindrical collar of securement device 132 and hemostasis valve 131 may include a deformable seal or gasket (e.g., a rubber washer) to both provide a seal between the parts and to provide backpressure tending to prevent peg 131a from easily moving within the slot of securement device 132. Moreover, a detent may be provided at the end points 132b of each slot in securement device 132 so that peg 131a will tend to remain in the locked state.

As already noted, in the example of FIGS. 1 and 2, the securement device 132 is a conventional Tuohy-Borst device in which a barrel 132c is rotated to vary the amount of resistance imposed on whatever object (e.g., catheter 122) is within the securement device 132. Again, as will be discussed in further detail below, any of the improved securement devices depicted and described with respect to FIGS. 3-9 may be substituted into the system shown in FIGS. 1 and 2. Moreover, as also noted above, in all cases, the securement devices of the present technology may be used in association with a repositioning sheath (e.g., as a part of or connecting to a hub of the repositioning sheath) or in association with an introducer sheath (e.g., as a part of or connecting to a hub of the introducer sheath).

FIG. 3 shows an enlarged view of a blood pump and repositioning sheath assembly using an exemplary button-actuated securement device, in accordance with aspects of the disclosure. Like FIG. 2, the example of FIG. 3 shows a portion of a blood pump and repositioning sheath assembly, oriented with its distal end to the left. All shared numbers between FIG. 3 and those of FIGS. 1 and 2 represent the same features. Thus, the catheter 122, repositioning sheath 126 (of which only proximal end 128 is identified), butterfly 130, hemostasis valve 131, and protective sleeve 136 all are as previously described. However, unlike FIG. 2, rather than showing a conventional Tuohy-Borst type securement device, the example of FIG. 3 employs a button-actuated securement device 200.

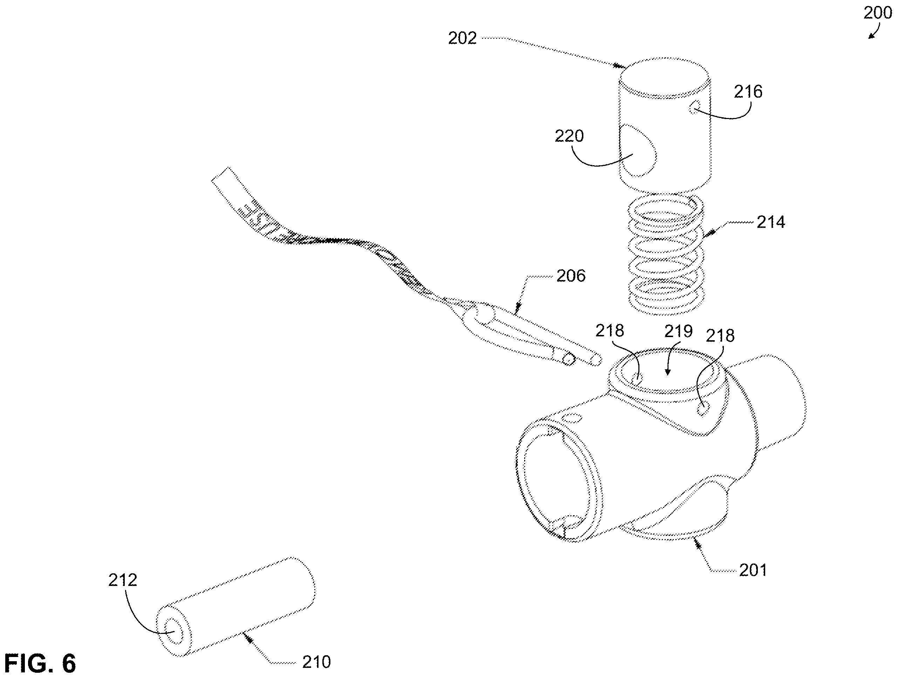

Securement device 200 comprises a body 201, which houses a button 202. The body 201 of securement device 200 includes two eyelets 204 (only one of which is visible in FIG. 3), each of which are configured to engage a peg 131a (not shown, but as described above) to achieve a snap-fit between hemostasis valve 131 and securement device 200. As will be described further below with respect to FIG. 6, button 202 is spring-loaded such that it will clamp down upon an internal flexible sleeve 210 (not visible in FIG. 3), and thus provide resistance to whatever object is inserted through the lumen of that flexible sleeve whenever the button 202 is not actively being pressed. To avoid placing sustained pressure on the flexible sleeve 210 prior to use (e.g., while securement device 200 is shelved in inventory), which may cause some amount of lasting deformation of the flexible sleeve, and/or to allow easier movement of the pump at the start of a procedure, the example securement device 200 includes a retention pin 206. As will be described further below with respect to FIG. 6, retention pin 206 can be inserted through bores in body 201 and button 202 to hold button 202 in a depressed state, so that the flexible sleeve 210 will not be subjected to pressure by button 202.

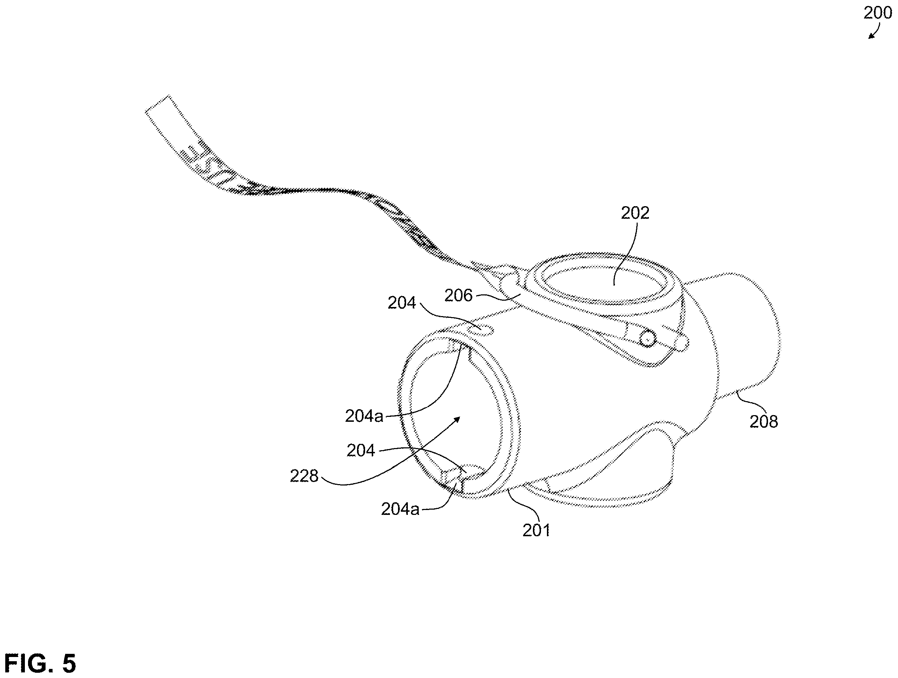

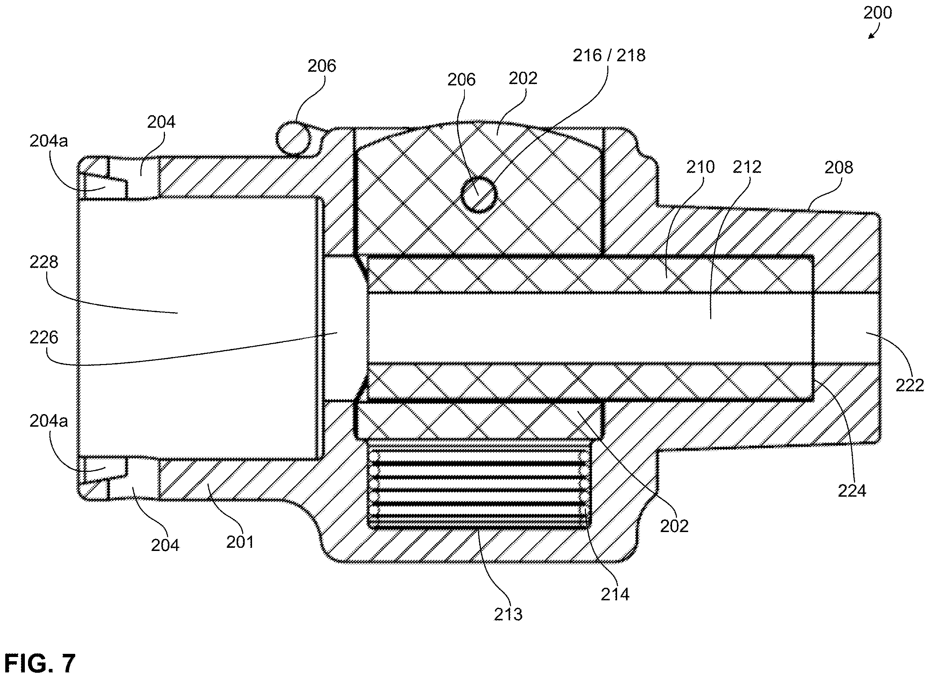

FIG. 4 shows an isometric view of a three-dimensional model of the exemplary button-actuated securing device of FIG. 3, and FIG. 5 shows a line-drawing thereof. As FIGS. 4 and 5 show the securing device 200 in isolation, the distal lumen 228 of body 201 can be seen in more detail. In particular, two slots 204a are provided which correspond to the diameter of eyelets 204. Slots 204a ramp up slightly in the proximal direction such that, when the cylindrical projection of hemostasis valve 131 is advanced into the cylindrical collar at the distal end of body 201, the resistance between pegs 131a and slots 204a will gradually increase until both pegs 131a "snap" into engagement with both eyelets 204. The ramping of slots 204a can also be seen in the cross-sectional view of FIG. 7.

The three-dimensional model of FIG. 4 also more clearly shows that body 201 has a proximal collar 208 that is configured to couple with protective sleeve 136. The coupling between proximal collar 208 and protective sleeve 136 may be accomplished by any suitable way. For example, protective sleeve 136 may be bonded to collar 208 or affixed thereto using adhesives, etc. In addition, protective sleeve 136 may be removably coupled to collar 208 using any suitable mechanical coupling, such as a ring mounted on collar 208 that can be screwed down or otherwise moved into engagement with another surface of body 201 in order to trap the end of protective sleeve 136.

The three-dimensional model and line-drawing of FIGS. 4 and 5 also show a retention pin 206 that is slightly different than what is shown in FIG. 3. As in FIG. 3, retention pin 206 is optional, and any suitable shape or configuration of retention pin may be employed.

FIG. 6 shows an exploded view of the exemplary button-actuated securing device of FIG. 3. In this exploded view, it can be seen that button 202 has two bores. Bore 216 is configured to allow passage of retention pin 206 through a portion of button 202. Body 201 has two identical or similarly sized bores 218. Accordingly, bores 216 and 218 allow retention pin 206 to be passed through both body 201 and button 202, thus holding button 202 in a depressed state.

Button 202 also has a second bore 220 that is sized to allow passage of flexible sleeve 210. Flexible sleeve 210 may be comprised of any suitable material such as silicone, etc. Flexible sleeve 210 has a lumen 212 that is sized to allow passage of whatever device is intended to be passed through securement device 200. Thus, if securement device 200 is used in the blood pump and repositioning sheath assembly such as those shown in FIGS. 1-3, lumen 212 may be sized to allow passage of catheter 122 when flexible sleeve 210 is in a relaxed, uncompressed state. The dimensions and materials used for flexible sleeve 210 may be chosen in part to provide a desired amount of friction between the lumen 212 and whatever medical device is intended to be passed through securement device 200, and/or to provide sufficient cushioning to the medical device to prevent damage to the medical device when the button 202 of the securement device 200 is not being pressed. For example, if securement device 200 is used in the blood pump and repositioning sheath assembly such as those shown in FIGS. 1-3, the flexible sleeve 210 may be configured by choosing a particular material, wall thickness, etc. to provide sufficient friction to the catheter 122 to prevent movement of the catheter 122 when the button 202 is not being pressed, while also avoiding crimping the catheter 122 and/or adversely impacting any electrical wires, purge lumens, flexible drive shafts, etc. which may pass through the catheter 122.

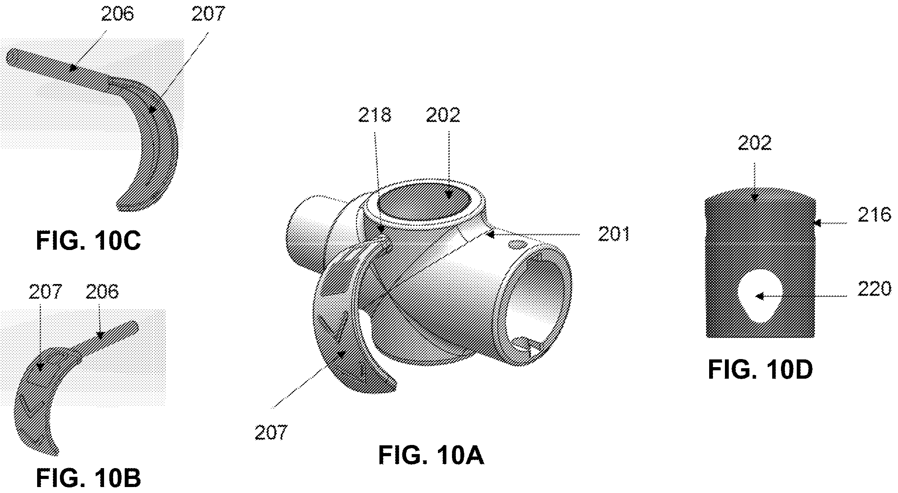

A different configuration for the retention pin is illustrated in FIGS. 10A-10D. As illustrated in FIG. 10A, FIG. 10B, and FIG. 10C, the retention pin 206 has a curved handle 207 that enables a user to remove the pin 206 simply by inserting their finger behind the handle 207 and removing the pin 206 from the bore 216 in button 202 and from the bore 218 in the body 201. As illustrated in FIG. 10D, the second bore 220 in the button 202 is tapered toward the lower portion of the button 202 received into the body 201. Therefore, the second bore 220 has a larger diameter at the top portion of the second bore 220 and a smaller diameter at the lower portion of the second bore 220, the "top" and "lower" portions of the button 202 being in relation to the "top" portion of the body 201 into which the button 202 is received. As described in detail above, this design facilitates the action of button on the catheter so that the catheter is held securely, but not crimped, when the button 202 is in the compressed (depressed) state.

As will be explained further below with respect to FIG. 7, body 201 includes a medial lumen 226 (not visible in FIG. 6) that is sized to retain flexible sleeve 210. In addition, body 201 includes a recess 213 (also not visible in FIG. 6) configured to retain spring 214 underneath button 202. Although spring 214 is depicted as a coil-spring, any suitable spring member may be substituted, such as a leaf spring, elastomeric element, living hinge, etc. When securement device 200 is assembled such that spring 214 is held within recess 213, and the flexible sleeve 210 is threaded through the bore 220 of button 202 and through the medial lumen 226 of body 201, spring 214 will be in compression, and thus providing a force tending to push button 202 upward and out of button cavity 219. This force will in turn cause bore 220 to provide a force tending to push flexible sleeve 210 upward, trapping it against the top of medial lumen 226. By selecting a spring 214 of sufficient strength, and a flexible sleeve 210 that is sufficiently flexible, this upward force on button 202 will cause flexible sleeve 210 to deform, and lumen 212 to pinch whatever object may be inserted through it (e.g., catheter 122), thus resisting movement of the object through lumen 212. Conversely, by depressing button 202, spring 214 will be further compressed, and the force on flexible sleeve 210 will be reduced or removed, allowing flexible sleeve 210 to return to a relaxed state in which lumen 212 will allow passage of whatever object is inserted through it (e.g., catheter 122). Either or both of the alternative retention pin design and the alternative second bore design described herein with respect to FIGS. 10A-10D may be combined with the other embodiments of the button-actuated securing device described herein.

FIG. 7 shows a cross-sectional profile view of the exemplary button-actuated securing device of FIG. 3. All reference numerals in common with FIGS. 3-6 identify the same features described above. As can be seen from the cross-sectional view of FIG. 7, body 201 has a proximal lumen 222 of substantially similar size to lumen 212, a medial lumen 226 of substantially similar size to the relaxed outer diameter of flexible sleeve 210, and a distal lumen 226 of substantially similar size to the cylindrical projection of hemostasis valve 131. FIG. 7 shows securement device 200 with retention pin 206 having been inserted through bores 216 and 218, and thus shows button 202 in a depressed state. As described above, once retention pin 206 is removed, spring 214 will begin to press upward on the bottom surface of button 202, which will in turn cause bore 220 of button 202 to press flexible sleeve 210 against the top of medial lumen 226, resulting in compression of the flexible sleeve 210 and its lumen 212.

FIG. 8 shows an enlarged view of a blood pump and repositioning sheath assembly using an exemplary button-actuated securement device, in accordance with aspects of the disclosure. In that regard, like FIGS. 2 and 3, the example of FIG. 8 shows a portion of a blood pump and repositioning sheath assembly, oriented with its distal end to the left. All shared numbers between FIG. 8 and those of FIGS. 1-3 represent the same features. Thus, the catheter 122, repositioning sheath 126 (and its proximal end 128), butterfly 130, hemostasis valve 131, and protective sleeve 136 all are as previously described. Like FIGS. 3-7, FIG. 8 also shows a securement device 300 which includes a button 302, and which is configured to restrict movement of an object inserted through it in the same way as described above with respect to FIGS. 3-7. However, unlike FIGS. 3-7, the securement device 300 of FIG. 8 is configured to be coupled to hemostasis valve using a bayonet connection similar to that which is described above with respect to FIG. 2.

In that regard, in FIG. 8, the distal end of body 301 of securement device 300 is configured such that it can be coupled to a proximal end of hemostasis valve 131 by pushing the two parts together and turning them relative to one another. As with the example of FIGS. 1 and 2, the distal portion of securement device 300 has a cylindrical collar with one or more slots, and the proximal portion of hemostasis valve 131 has a cylindrical projection with one or more pegs 131a. When the cylindrical collar of securement device 300 is advanced over the cylindrical projection of hemostasis valve 131, each peg 131a will enter one of the slots in securement device 300 at an entrance point 304a. Then, by rotating the securement device 300 relative to hemostasis valve 131, each peg 131a will move toward an end point 304b of the slot, thus preventing securement device 300 from being pulled away from hemostasis valve 131 (without first rotating the two components in the opposite direction). In addition, the internal interface between the cylindrical collar of securement device 300 and hemostasis valve 131 may include a deformable seal or gasket (e.g., a rubber washer) to both provide a seal between the parts and to provide backpressure tending to prevent peg 131a from easily moving within the slot of securement device 300. Moreover, a detent may be provided at the end points 304b of each slot in securement device 300 so that peg 131a will tend to remain in the locked state.

In addition, in the example of FIG. 8, protective sleeve 136 extends over button 302 to prevent the possibility of fluids, microorganisms, etc. from entering securement device 300 around the edges of button 302, and thus contaminating catheter 122 where it passes within securement device 300. Securement device 300 has a retaining ring 306 configured to trap the end of protective sleeve 136 against an opposing surface of body 301. As described above, this trapping arrangement may be accomplished in any suitable way. Likewise, in some aspects of the technology, protective sleeve 136 may be bonded directly to ring 306 and/or body 301, or affixed thereto using an adhesive.

FIG. 9 shows an enlarged view of a blood pump and repositioning sheath assembly using an exemplary button-actuated securement device, in accordance with aspects of the disclosure. Here as well, the example of FIG. 8 shows a portion of a blood pump and repositioning sheath assembly, oriented with its distal end to the left. All shared numbers between FIG. 9 and those of FIGS. 1-3 represent the same features. Thus, the catheter 122, repositioning sheath 126 (and its distal end 124 and proximal end 128), butterfly 130, and protective sleeve 136 all are as previously described. FIG. 9 also shows a securement device 400 which includes a button 402, and which is configured to restrict movement of an object inserted through it in the same way as described above with respect to FIGS. 3-7. However, unlike FIGS. 1-8, the securement device 400 of FIG. 9 includes a hemostasis valve (not shown) within it. As such, the portion of the blood pump and repositioning sheath assembly shown in FIG. 9 does not require a separate hemostasis valve 131, and therefore the body 401 of securement device 400 does not require any features to enable a coupling (e.g., a bayonet connection, snap-fit connection, etc.) between it and hemostasis valve 131. In that regard, in some aspects of the technology, securement device 400 may be a modular unit that attaches directly to butterfly 130 using any suitable coupling arrangement. However, in other aspects of the technology, securement device 400 may be integral to the hub of the repositioning sheath assembly, and thus repositioning sheath 126, butterfly 130, and securement device 400 may be part of a single repositioning sheath assembly. Likewise, as mentioned above, securement device 400 may also be configured to work with or be an integral part of an introducer sheath assembly (e.g., introducer sheath assembly 150).

From the foregoing and with reference to the various figures, those skilled in the art will appreciate that certain modifications can also be made to the present disclosure without departing from the scope of the same. While several aspects of the disclosure have been shown in the figures, it is not intended that the disclosure be limited thereto, as it is intended that the disclosure be as broad in scope as the art will allow and that the specification be read likewise. Therefore, the above description should not be construed as limiting, but merely as exemplifications of particular aspects of the present technology.

## Claims

1. A device (131; 200; 300; 400) for securing an intravenous or intra-arterial medical device (122), comprising:
a flexible sleeve (210) having a first lumen (212) configured to receive at least a portion of the intravenous medical device (122),
a button (202; 302; 402) having a bore (220) configured to receive at least a portion of the flexible sleeve (210),
a spring element (214), and
a housing (201; 301; 401) comprising a second lumen (226) configured to house the flexible sleeve (210) and a cavity (219) configured to house the button (202; 302; 402) and the spring element (214),
wherein the spring element (214) is configured to exert a force on the button (202; 302; 402) tending to cause the bore (220) to compress the flexible sleeve (210) unless the button (202; 302; 402) is being held in a depressed state,
**characterized in that** the housing (201; 301; 401) comprises either a hemostasis valve (131) or at least one slot configured to accept at least one peg (131a) of a hemostasis valve (131) when the housing (201; 301; 401) is coupled with the hemostasis valve (131).

2. The device of claim 1, wherein the housing (201; 301; 401) comprises at least one protrusion (130) with at least one eyelet (130a - 130d), the at least one protrusion (130) configured to enable the housing to be sutured to a patient's skin.

3. The device of claim 1 or 2, wherein the at least one slot and the at least one peg (131a) comprise a bayonet connection.

4. The device of claim 3, wherein the at least one slot further comprises at least one eyelet (204) configured to engage with the at least one peg (131a).

5. The device of claim 3, wherein the at least one slot further comprises at least one detent configured to engage with the at least one peg (131a).

6. The device of one of claims 1 to 5, wherein the housing (201; 301; 401) further comprises a seal configured to deform when the housing (201; 301; 401) is coupled with the hemostasis valve (131).

7. A sheath assembly (100), comprising:
a sheath body (152) configured for insertion into a patient's vasculature and for receiving at least a portion of an intravenous or intra-arterial medical device (122)
a sheath hub (154) coupled to a proximal end of the sheath body (152); and
a device (131; 200; 300; 400) according to any of claims 1 to 6 for securing the intravenous or intra-arterial medical device (122), said device being integral with the sheath hub or configured to couple with the sheath hub.

8. The sheath assembly of claim 7, wherein the sheath hub (154) comprises at least one protrusion with at least one eyelet, the at least one protrusion configured to enable the sheath hub (154) to be sutured to a patient's skin.

9. The sheath assembly of claim 7 or 8, wherein the sheath hub (154) further comprises a hemostasis valve.

## Patentansprüche

1. Vorrichtung (131; 200; 300; 400) zum Sichern einer intravenösen oder intraarteriellen medizinischen Vorrichtung (122), umfassend:
eine flexible Hülse (210), die ein erstes Lumen (212) hat, das zum Aufnehmen mindestens einen Abschnitts der intravenösen medizinischen Vorrichtung (122) konfiguriert ist,
einen Knopf (202; 302; 402), der eine Bohrung (220) hat, die zum Aufnehmen mindestens eines Abschnitts der flexiblen Hülse (210) konfiguriert ist,
ein Federelement (214), und
ein Gehäuse (201; 301; 401) mit einem zweiten Lumen (226), das zum Unterbringen der flexiblen Hülse (210) konfiguriert ist, und einen Hohlraum (219), der zum Unterbringen des Knopfes (202; 302; 402) und des Federelements (214) konfiguriert ist,
wobei das Federelement (214) konfiguriert ist, eine Kraft auf den Knopf (202; 302; 402) auszuüben, die dazu neigt, die Bohrung (220) zum Zusammendrücken der flexiblen Hülse (210) zu bringen, es sei denn, der Knopf (202; 302; 402) wird in einem gedrückten Zustand gehalten,
**dadurch gekennzeichnet, dass** das Gehäuse (201; 301; 401) ein Hämostaseventil (131) oder mindestens einen Schlitz aufweist, der konfiguriert ist, mindestens einen Zapfen (131a) eines Hämostaseventils (131) aufzunehmen, wenn das Gehäuse (201; 301; 401) mit dem Hämostaseventil (131) gekoppelt ist.

2. Vorrichtung nach Anspruch 1, wobei das Gehäuse (201; 301; 401) mindestens einen Vorsprung (130) mit mindestens einer Öse (130a - 130d) aufweist, wobei der mindestens eine Vorsprung (130) konfiguriert ist, das Nähen des Gehäuses an die Haut eines Patienten zu ermöglichen.

3. Vorrichtung nach Anspruch 1 oder 2, wobei der mindestens eine Schlitz und der mindestens eine Zapfen(131a) einen Bajonettanschluss aufweisen.

4. Vorrichtung nach Anspruch 3, wobei der mindestens eine Schlitz weiter mindestens eine Öse (204) aufweist, die zum Eingreifen mit dem mindestens einen Zapfen (131a) konfiguriert ist.

5. Vorrichtung nach Anspruch 3, wobei der mindestens eine Schlitz weiter mindestens eine Arretierung aufweist, die zum Eingreifen mit dem mindestens einen Zapfen (131a) konfiguriert ist.

6. Vorrichtung nach Anspruch 6, wobei das Gehäuse (201; 301; 401) weiter eine Dichtung aufweist, die konfiguriert ist, zu deformieren, wenn das Gehäuse (201; 301; 401) mit dem Hämostaseventil (131) gekoppelt ist.

7. Hülsenanordnung (100), umfassend:
einen Hülsenkörper (152), der zum Einführen in das Gefäßsystem eines Patienten und zum Aufnehmen mindestens eines Abschnitts einer intravenösen oder intraarteriellen Vorrichtung (122) konfiguriert ist,
eine Hülsennabe (154), die mit einem proximalen Ende des Hülsenkörpers (152) gekoppelt ist; und
eine Vorrichtung (131, 200; 300; 400) gemäß einem der Ansprüche 1 bis 6 zum Sichern der intravenösen oder intraarteriellen medizinischen Vorrichtung (122), wobei die Vorrichtung integral mit der Hülsennabe ausgebildet ist oder konfiguriert ist, mit der Hülsennabe zu koppeln.

8. Hülsenanordnung nach Anspruch 7, wobei die Hülsennabe (154) mindestens einen Vorsprung mit mindestens einer Öse umfasst, wobei der mindestens eine Vorsprung konfiguriert ist, das Nähen der Hülsennabe an die Haut eines Patienten zu ermöglichen.

9. Hülsenanordnung nach Anspruch 7 oder 8, wobei die Hülsennabe (154) weiter ein Hämostaseventil umfasst.

## Revendications

1. Dispositif (131; 200; 300; 400) destiné à arrimer un dispositif médical intraveineux ou intra-artériel (122), comprenant :
un manchon flexible (210) présentant une première lumière (212) configurée pour recevoir au moins une portion du dispositif médical intraveineux (122) ;
un bouton (202; 302; 402) présentant un alésage (220) configuré pour recevoir au moins une portion du manchon flexible (210),
un élément à ressort (214), et
un logement (201; 301; 401) comprenant une seconde lumière (226) configurée pour loger le manchon flexible (210) et une cavité (219) configurée pour loger le bouton (202; 302; 402) et l'élément à ressort (214) ;
dans lequel l'élément à ressort (214) est configuré pour exercer une force sur le bouton (202; 302; 402) tendant à faire en sorte que l'alésage (220) comprime le manchon flexible (210) sauf si le bouton (202; 302; 402) est maintenu dans un état enfoncé ;
**caractérisé en ce que** le logement (201; 301; 401) comprend soit une valve hémostatique (131), soit au moins une fente configurée pour accepter au moins un tenon (131a) d'une valve hémostatique (131) lorsque le logement (201; 301; 401) est accouplé à la valve hémostatique (131).

2. Dispositif selon la revendication 1, dans lequel le logement (201; 301; 401) comprend au moins une saillie (130) avec au moins un œillet (130a-130d), la au moins une saillie (130) étant configurée pour permettre de suturer le logement sur la peau d'un patient.

3. Dispositif selon la revendication 1 ou 2, dans lequel la au moins une fente et le au moins un tenon (131a) comprend un raccord à baïonnette.

4. Dispositif selon la revendication 3, dans lequel la au moins une fente comprend en outre au moins un œillet (204) configuré pour venir en prise avec le au moins un tenon (131a).

5. Dispositif selon la revendication 3, dans lequel la au moins une fente comprend en outre au moins un encliquetage configuré pour venir en prise avec le au moins un tenon (131a).

6. Dispositif (1) selon l'une quelconque des revendications 1 à 5, dans lequel le logement (201; 301; 401) comprend en outre un moyen d'étanchéité configuré pour se déformer lorsque le logement (201; 301; 401) est accouplé avec la valve hémostatique (131).

7. Ensemble de gaine (100), comprenant :
un corps de gaine (152) configuré pour une introduction dans la vasculature d'un patient et pour recevoir au moins une portion d'un dispositif médical intraveineux ou intra-artériel (122) ;
un moyen de gaine (154) accouplé à une extrémité proximale du corps de gaine (152), et
un dispositif (131; 200; 300; 400) selon l'une quelconque des revendications 1 à 6 pour arrimer le dispositif médical intraveineux ou intra-artériel (122), ledit dispositif étant d'un seul tenant avec le moyeu de gaine ou configuré pour s'accoupler avec le moyeu de gaine.

8. Ensemble de gaine selon la revendication 7, dans lequel le moyeu de gaine (154) comprend au moins une saillie avec au moins un œillet, et la au moins une saillie étant configurée pour permettre de suturer le moyeu de gaine (154) sur la peau d'un patient.

9. Ensemble de gaine selon la revendication 7 ou 8, dans lequel le moyeu de gaine (154) comprend en outre une valve hémostatique.
